(19) Europäisches Patentamt
European Patent Office
Office européen des brevets

(11) **EP 1 885 419 B1**

(12) **EUROPÄISCHE PATENTSCHRIFT**

(45) Veröffentlichungstag und Bekanntmachung des Hinweises auf die Patenterteilung:
**18.01.2012 Patentblatt 2012/03**

(21) Anmeldenummer: 06723307.2

(22) Anmeldetag: **09.03.2006**

(51) Int Cl.:
*A61M 11/06* (2006.01)      *A61M 15/00* (2006.01)

(86) Internationale Anmeldenummer:
**PCT/EP2006/002154**

(87) Internationale Veröffentlichungsnummer:
**WO 2006/094796 (14.09.2006 Gazette 2006/37)**

(54) **INHALATOR MIT EINEM MISCHKANAL ZUM ERZEUGEN EINES ZU INHALIERENDEN AEROSOLS**

INHALER WITH A MIXING CHANNEL FOR PRODUCING AN AEROSOL TO BE INHALED

INHALATEUR DOTÉ D'UN CANAL MÉLANGEUR DESTINÉ À LA PRODUCTION D'UN AÉROSOL À INHALER

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **10.03.2005 DE 102005010965**

(43) Veröffentlichungstag der Anmeldung:
**13.02.2008 Patentblatt 2008/07**

(73) Patentinhaber: **Medspray Xmems BV
7521 PV Enschede (NL)**

(72) Erfinder:
• **WISSINK, Jeroen Mathijn
7512 DW Enschede (NL)**
• **NIJDAM, Wietze
7201 JE Zutphen (NL)**
• **HESKAMP, Iwan Rutger
7521 PT Enschede (NL)**
• **PADBERG, Marc, Herman
7511 RM Enschede (NL)**

(74) Vertreter: **Knoblauch, Andreas
Patentanwälte Dr. Knoblauch
Schlosserstrasse 23
60322 Frankfurt am Main (DE)**

(56) Entgegenhaltungen:
**DE-A1- 4 306 458      GB-A- 1 227 333
US-A- 4 592 348      US-B1- 6 447 816**

**Beschreibung**

Die Erfindung bezieht sich auf einen Inhalator mit einem Mischkanal zum Erzeugen eines zu inhalierenden Aerosols, wobei der Mischkanal aufweist: eine in den Mund einer Person einzuführende Auslaßöffnung an seinem einen Ende zum Inhalieren des erzeugten Aerosols; wenigstens eine Einlaßöffnung an seinem anderen Ende zum Einsaugen von Luft in den Mischkanal und wenigstens einen zwischen der Einlaßöffnung und der Auslaßöffnung liegenden, einen Teil der Kanalwand bildenden Einspritzbereich, wobei der Einspritzbereich wenigstens eine Düsenöffnung zum Einspritzen einer Flüssigkeit, insbesondere eines flüssigen Medikaments, in Form eines Strahls pro Düsenöffnung aus dispersen Tröpfchen in den Mischkanal aufweist, um die Tröpfchen durch den eingesaugten Luftstrom mitzureißen und in einem Endbereich zwischen Einspritzbereich und Auslaßöffnung getrennt zu halten, nachdem sich die Tröpfchen mit dem Luftstrom zu dem Aerosol vermischt haben.

Bei einem aus der internationalen Patentanmeldung WO 02/18058 A1 (Fig. 16) bekannten Mischkanal dieser Art ist der Einspritzbereich durch eine Düsenplatte gebildet, die mit der Wand bzw. Längsmittelachse des Mischkanals einen Winkel von +10° bis +90° einschließt. Bei dieser Lage der Düsenplatte im Mischkanal ergeben sich Luftwirbel an den Rändern der Düsenplatte beim Inhalieren der Luft durch den Mischkanal hindurch. Diese Wirbel verringern die Atemluftgeschwindigkeit und verhindern, daß die sich nach dem Austreten des zunächst kontinuierlichen Strahls bildenden, sehr kleinen Tröpfchen bei der Vermischung mit der Luft getrennt bleiben. Sie können zu größeren und nicht mehr monodispersen Tröpfchen koagulieren und dann nicht an die gewünschte Stelle, insbesondere bis in die richtigen Kanäle der Lunge, eindringen und sich teilweise an der Kanalwand niederschlagen. Dies vermindert die Wirksamkeit der Flüssigkeit bzw. des Medikaments.

Dokument DE 4306458 offenbart einen Vernebler für therapeutische Zwecke. Aus diesem Dokument sind alle Merkmale des Oberbegriffs des Anspruchs 1 entnehmbar.

Der Erfindung liegt die Aufgabe zugrunde, einen Inhalator der eingangs erwähnten Art so zu verbessern, daß die im Aerosol enthaltenen Tröpfchen der Flüssigkeit, insbesondere des flüssigen Medikaments, bei der Benutzung des Inhalators getrennt bis in den Mund, Rachen und nötigenfalls bis in die feinsten Verästelungen der Lunge eindringen können, ohne sich an der Kanalwand niederzuschlagen.

Erfindungsgemäß ist diese Aufgabe durch die Merkmale des Anspruchs 1 gelöst. Die Innenseite des Einspritzbereichs fluchtet zumindest mit dem ihr einlaßseitig benachbarten Teil des mittleren Oberflächenverlaufs der Innenseite der Kanalwand weitgehend , wobei ein etwaiger Höhenunterschied zwischen der Innenseite des Einspritzbereichs und dem ihr benachbarten Teil des mittleren Oberflächenverlaufs der Innenseite der Kanalwand kleiner als 1 mm ist oder höchstens 100 $\mu$m oder höchstens 20 $\mu$m beträgt.

Bei dieser Lösung werden im Mischkanal hervorstehende Kanten weitgehend vermieden, bei deren Umströmung durch die inhalierte Luft Wirbel entstehen würden, die dazu führen können, daß die Flüssigkeitströpfchen koagulieren oder sich auf der Kanalwand niederschlagen.

Neben der Verteilung der Tröpfchengröße beeinflussen auch die Inhalationsgeschwindigkeit und das Inhalationsvolumen das Niederschlagen des Aerosols im Atmungssystem. Die Inhalationsgeschwindigkeit und das Inhalationsvolumen hängen jedoch auch von der inhalierenden Person, also von den Betriebsbedingungen, und nicht nur von Konstruktionsparametern des Mischkanals ab. Dennoch wird das Aerosol durch einen Volumenstrom der Luft beim Inhalieren erzeugt, der höher als die normale Geschwindigkeit der Atemluft liegt. Dies kann dadurch erreicht werden, daß der Durchtrittsquerschnitt des Mischkanals bis zum Einspritzbereich konvergiert oder mindestens bis zu diesem konstant ist. Dabei ist der Durchtrittsquerschnitt des Kanals an der engsten Stelle kleiner als die normale Öffnungsweite des Mundes, bei normaler Einatmung durch den Mund. Dies trägt zur Erhöhung der Strömungsgeschwindigkeit des Aerosols durch den Mischkanal und dazu bei, daß einerseits die Strahlen, wenn es mehrere sind, in monodisperse Tröpfchen getrennt werden und andererseits die Tröpfchen des oder jedes Strahls voneinander getrennt bleiben, so daß der monodisperse Charakter des Aerosols weitgehend erhalten bleibt. Die Beibehaltung des monodispersen Zustands der Tröpfchen kann durch bestimmte Kanaleigenschaften und -abmessungen erreicht werden.

Sodann kann dafür gesorgt sein, daß die Kanalwand in Luftströmungsrichtung linear divergiert oder konvergiert und sich der Flächeninhalt A(x) des Durchtrittsquerschnitts des Mischkanals, unabhängig von der Kontur des Durchtrittsquerschnitts, mit dem Abstand x von der Einlaßöffnung oder von dem kleinsten Durchtrittsquerschnitt im Einspritzbereich ändert und die Änderung dA(x)/dx an der Stelle x zwischen $-c_1\sqrt{A(x)}$ und 0 oder zwischen $c_2\sqrt{A(x)}$ und 0 liegt und $c_1$ = 15,35 oder 4,22 und $c_2$ = 1,58 oder 0,88 oder 0,31 ist. Dies stellt sicher, daß sich der Luftstrom, auch bei sich über die Länge des Mischkanals ändernder Form des Durchtrittsquerschnitts, nicht von der Kanalwand löst und frei von Wirbeln bleibt und die Atemluftgeschwindigkeit minimiert wird. Unterhalb von $c_2$ = 1,53, insbesondere unterhalb von $c_2$ = 0,31, besteht nicht die Gefahr, daß sich die Luft von der Kanalwand ablöst, so daß Wirbel entstehen würden. In der Luftströmung werden die Tröpfchen voneinander und von der Kanalwand getrennt gehalten. Sie behalten daher ihre ursprüngliche geringe Größe mit einem Durchmesser von etwa 1 bis 10 $\mu$m bei, wobei der Flüssigkeitsstrahl beim Austritt aus der Düsenöffnung zunächst kontinuierlich ist, und zwar mit einem Durchmesser von etwa 0,5 bis 5 $\mu$m, entsprechend dem Durchmesser der Düsenöffnung, bevor er in geringem Abstand von der Düsenöffnung in monodisperse Tröpfchen (gleicher Größe) übergeht. Die Tröpfchen koagulieren kaum, sondern bleiben weiterhin bis zum Eintritt in die feinen Lungenkanäle oder Bronchien weitgehend monodispers, ohne sich an der Kanalwand niederzuschlagen. Dies ermöglicht eine bessere Aufnahme des Medikaments in der Lunge, und zwar etwa 30 % bis fast 100 %, statt bisher 0 bis 30 %. So würde ein größerer Anteil kleinerer Tröpfchen mit einem Durchmesser von 2 $\mu$m auch die Lungenbläschen, wenn die Größenverteilung der Tröpfchen monodispers ist, und der größeren Tröpfchen von 5 $\mu$m die Bronchien erreichen. Vorzugsweise ist die geometrische Standardabweichung (GSA) des Durchmessers der Tröpfchen im Aerosol so abgestimmt und optimiert, daß der Massenmedian des aerodynamischen Durchmessers (MMAD) der Tröpfchen des Aerosols im Bereich von 1 bis 5 $\mu$m oder 1 bis wenigstens 10 $\mu$m und die GSA im Idealfall bei 1,0 liegt.

Die Kanalwand kann in einer Ausführungsform so geformt sein, daß der Mischkanal von der Einlaßöffnung zum Einspritzbereich konvergiert und dahinter wieder divergiert. Durch das Divergieren des Kanals wird die im Einspritzbereich zunächst höhere Strömungsgeschwindigkeit des Aerosols wieder verringert, so daß sie sich weitgehend an die normale Einatmungsgeschwindigkeit anpaßt und die Tröpfchen sich nicht wesentlich im Mundraum und Hals niederschlagen. Dies kann alternativ auch durch das Konvergieren oder den konstanten Durchtrittsquerschnitt erreicht werden, wobei im letzten Fall der Mund als divergierender Kanal wirkt.

Eine weitere Möglichkeit besteht darin, daß die Kanalwand bei horizontaler Haltung des Mischkanals zumindest oberhalb und unterhalb seiner Längsmittelachse in Bezug auf diese in einem sich über den Einspritzbereich hinweg erstreckenden Abschnitt konvergiert und stromunterhalb des konvergierenden Abschnitts divergiert.

Ferner ist eine Ausführungsform möglich, bei der die Kanalwand bei horizontaler Haltung des Mischkanals zumindest oberhalb und unterhalb seiner Längsmittelachse von der Einlaßöffnung bis zum Einspritzbereich trompetenartig konvergiert.

Am günstigsten ist es, wenn die Axialschnittkontur der Innenseite des trompetenartig konvergierenden Abschnitts der Kanalwand zumindest oberhalb und unterhalb der Längsmittelachse dem Verlauf einer Parabel dritten Grades entspricht.

Besonders günstig ist eine Ausführungsform, bei der der Durchtrittsquerschnitt des Mischkanals in aufeinanderfolgenden Längsabschnitten von einer Rechteckform an der Einlaßöffnung zu einer Rechteckform mit abgerundeten Ecken über den Einspritzbereich hinweg stetig abnimmt und daran anschließend über Rechteckformen mit abgerundeten Ecken und nach außen gewölbten Seiten in eine Kreisform übergeht.

Vorzugsweise ist dafür gesorgt, daß der Strahl unter einem Winkel $\alpha$ zu einer Tangente an den Einspritzbereich zwischen 10° und 170°, vorzugsweise zwischen 10° und 90° oder zwischen 90° und 170°, aus dem Einspritzbereich austritt, und der Strahl anfänglich bei $\alpha$ < 90° zur Auslaßöffnung und bei $\alpha$ > 90° zur Einlaßöffnung hin geneigt ist.

Die Länge eines sich an den Einspritzbereich in Axialrichtung des Mischkanals anschließenden Mischbereichs kann 1 bis 50 mm betragen.

Die Länge eines sich an den Mischbereich anschließenden Endbereichs kann das 2- bis 3-fache der Länge des Mischbereichs betragen.

Der Flächeninhalt des Durchtrittsquerschnitts des Mischkanals am Ende eines sich bis zum Einspritzbereich erstreckenden Einlaßbereichs sollte 1 bis 1000 mm$^2$ oder 5 bis 100 mm$^2$, vorzugsweise 10 bis 20 mm$^2$, betragen.

Vorzugsweise ist der Einspritzbereich durch eine Düsenplatte gebildet. Dadurch ist es möglich, den Einspritzbereich durch entsprechende Ausgestaltung der Düsenplatte auf einfache Weise separat optimal zu formen, und zwar unabhängig vom Material des Mischkanals. Insbesondere ist es auf einfache Weise möglich, die Düsenöffnung(en) günstig zu gestalten.

So ist es möglich, wenn der Mischkanal Kunststoff aufweist, um ihn auf einfache Weise zu formen, es sich aber als schwierig erweist, die Düsenöffnung(en) in diesem Material hinreichend klein auszubilden, die Düsenplatte aus einem Material, vorzugsweise mit Siliciumnitrid beschichtetes Silicium, herzustellen, in dem sich die Düsenöffnungen sehr klein, etwa mit einem Durchmesser von 0,5 bis 5 $\mu$m, ausbilden lassen und bei dem es auch möglich ist, daß die (anfängliche) Richtung des Strahlaustritts aus der Kanalwand den Winkel $\alpha$ mit der Tangente an der Strahlaustrittsstelle einschließt. Außerdem kann der Kunststoff des Mischkanals einen antibakteriellen und/oder elektrisch leitfähigen Zusatz oder Belag gegen eine elektrostatische Aufladung aufweisen. Der Zusatz kann aus Metall-, Kohle- oder Graphitteilchen oder einem leitfähigen Polymer bestehen. Der Belag kann aus Metall bestehen.

Sodann kann die Oberfläche der Innenseite der Kanalwand mindestens teilweise mikrostrukturiert sein, z.B. durch eine Oberflächenbehandlung, einen Oberflächenbelag oder durch entsprechende Formgebung in einem Formwerkzeug. Dies ergibt eine kleinere Reibung zwischen Luftstrom und Kanalwand als bei einer glatten Wand. Der Luftstrom wird dadurch erhöht.

Eine Koagulation der Tröpfchen kann durch maximierte Zugkräfte verhindert werden, die durch das Luftströmungsprofil und die Luftströmungsgeschwindigkeit auf die Tröpfchen ausgeübt werden. So kann an oder vor der Einlaßöffnung ein Luftströmungswiderstand angeordnet sein. Dieser kann eine Lochplatte mit wenigstens einem Loch sein. Der Grund ist der: Da der Strömungswiderstand des Mischkanals optimiert ist, kann eine zu hohe Strömungsgeschwindigkeit auftreten, wenn ein Patient normal mit 2 bis 4 kPa saugt. Wenn ein Luftwiderstand vorgeschaltet ist, wird die Luftströmungsgeschwindigkeit begrenzt. So hat insbesondere eine Lochplatte einen nichtlinearen Einfluß auf die Luftströmungsgeschwindigkeit. Ein Loch in der Platte, insbesondere ein rundes, kurzes Loch, ergibt einen überproportionalen, insbesondere quadratischen, Zusammenhang zwischen dem Druck (Unterdruck beim Saugen) und der Luftströmungsgeschwindigkeit. Dies hat zur Folge, daß bei geringer Saugkraft die Strömungsgeschwindigkeit kaum verringert wird. Wenn der Patient dagegen mit maximaler Kraft saugt, ergibt sich eine kleinere Strömungsgeschwindigkeit als ohne Luftwiderstand. Dies gibt dem Patienten ein besseres Gefühl beim Ansaugen. Er kann die Luft über längere Zeit gleichmäßig ansaugen.

Vorzugsweise ist dafür gesorgt, daß die Dosiereinheit eine Kolben-Zylinder-Anordnung zur Ausübung eines Drucks auf die in den Mischkanal einzuspritzende Flüssigkeit und ein durch einen manuellen Druck bewegbares Betätigungsteil aufweist, dessen Betätigungsweg über eine federnde Vorrichtung in eine Relativbewegung zwischen dem Kolben und dem Zylinder der Kolben-Zylinder-Anordnung umwandelbar ist. Bei dieser Ausbildung hängen der auf die Flüssigkeit ausgeübte Druck und ihre Geschwindigkeit beim Einspritzen sowie die Dauer der Einspritzung in den Mischkanal im wesentlichen nur von der Kraft der federnden Vorrichtung ab, die an die zur Einhaltung einer gewünschten Inhalationsdauer von etwa 0,5 bis 10 Sekunden und der entsprechenden Strömungsgeschwindigkeit des Strahls weitgehend angepaßt werden kann. Der Benutzer hat somit weniger Einfluß auf den Betätigungsdruck, was die Handhabung des Inhalators erleichtert.

Eine besonders geeignete Ausgestaltung des Inhalators kann dann darin bestehen, daß der Zylinder in einer Verschlußeinrichtung eines die Flüssigkeit enthaltenden Behälters dicht eingesetzt ist und in den Behälter ragt, daß eine Kolbenstange des Kolbens durch die Verschlußeinrichtung hindurchgeführt ist, daß der Kolben und die Kolbenstange von einem bis zu dem Einspritzbereich führenden Auslaßkanal durchsetzt sind, daß die Kolbenstange in einem die Verschlußeinrichtung verschiebbar aufnehmenden Gehäuse befestigt ist, daß der Betätigungsweg des Betätigungsteils über die federnde Vorrichtung gegen die Kraft einer weiteren federnden Vorrichtung auf die Verschlußeinrichtung übertragbar ist, so daß die Verschlußeinrichtung relativ zum Kolben verschoben wird, daß der Druckraum des Zylinders im unbetätigten Zustand des Betätigungsteils über wenigstens eine Öffnung in der Wand des Zylinders und über eine Ventilanordnung mit dem Innenraum des Behälters in Verbindung steht und daß die Öffnung(en) des Zylinders und die Ventilanordnung, nach Überfahren der Öffnung(en) des Zylinders durch den Kolben, während der Betätigung des Betätigungsteils gesperrt sind.

Die Erfindung und ihre Weiterbildungen werden nachstehend anhand von Zeichnungen bevorzugter Ausführungsbeispiele näher beschrieben. Darin stellen dar:

Fig. 1　　eine schematische Seitenansicht eines Inhalators zum Erzeugen eines zu inhalierenden Aerosols mit einem erfindungsgemäßen Mischkanal, der als Mundstück in den Mund eines Patienten zur Behandlung seiner Lunge eingeführt wird,

Fig. 2　　eine schematische, vergrößerte Ansicht eines Vertikalschnitts durch die Längsmittelachse eines Ausführungsbeispiels eines erfindungsgemäßen Mischkanals,

Fig. 3　　eine Ansicht eines Vertikalschnitts durch die Längsmittelachse eines zweiten Ausführungsbeispiels eines erfindungsgemäßen Mischkanals,

Fig. 4　　eine Ansicht eines Vertikalschnitts durch die Längsmittelachse eines dritten Ausführungsbeispiels eines erfindungsgemäßen Mischkanals,

Fig. 5　　eine Ansicht eines Vertikalschnitts durch die Längsmittelachse eines vierten Ausführungsbeispiels eines erfindungsgemäßen Mischkanals,

Fig. 6　　eine Ansicht eines Vertikalschnitts von drei gegenüber dem Mischkanal nach Fig. 2 abgewandelten Ausführungsbeispielen in derselben Darstellung eines erfindungsgemäßen Mischkanals,

Fig. 7　　eine Ansicht eines Vertikalschnitts durch die Längsmittelachse eines achten Ausführungsbeispiels eines erfindungsgemäßen Mischkanals,

Fig. 8　　eine Ansicht eines Vertikalschnitts durch die Längsmittelachse eines neunten Ausführungsbeispiels eines erfindungsgemäßen Mischkanals,

Fig. 9　　eine Ansicht eines Vertikalschnitts durch die Längsmittelachse eines zehnten Ausführungsbeispiels eines erfindungsgemäßen Mischkanals,

Fig. 10　　eine Ansicht eines Vertikalschnitts durch die Längsmittelachse eines elften Ausführungsbeispiels eines erfindungsgemäßen Mischkanals,

Fig. 11　　eine schematische, perspektivische Darstellung eines Ausführungsbeispiels eines erfindungsgemäßen Mischkanals zur Erläuterung der möglichen Änderung des Flächeninhalts des Durchtrittsquerschnitts eines Ausführungsbeispiels des erfindungsgemäßen Mischkanals,

Fig. 12　　ein Kurvendiagramm der Abhängigkeit der Änderung des Flächeninhalts A eines Ausführungsbeispiels eines erfindungsgemäßen Mischkanals vom Abstand x von dem kleinsten Durchtrittsquerschnitt eines erfindungsgemäßen Mischkanals,

| Fig. 13 | schematische Darstellungen verschiedener Formen a bis e des Durchtrittsquerschnitts eines bevorzugten Ausführungsbeispiels eines erfindungsgemäßen Mischkanals nach Fig. 14, |
|---|---|
| Fig. 14 | eine schematische Ansicht des Vertikalschnitts des bevorzugten Ausführungsbeispiels der Erfindung, |
| Fig. 15 | eine gegenüber der nach Fig. 14 um 90° um die Längsmittelachse gedrehte Ansicht des Vertikalschnitts des Mischkanals nach Fig. 14, |
| Fig. 16 bis 19 | Ausschnitte einer Kanalwand weiterer Ausführungsbeispiele erfindungsgemäßer Mischkanäle, |
| Fig. 20 | einen vergrößerten Ausschnitt der unteren Kanalwand des Mischkanals, in die ein Einspritzbereich in Form einer Düsenplatte nicht genau passend eingesetzt ist, |
| Fig. 21 | einen vergrößerten Ausschnitt der unteren Kanalwand, deren Oberfläche außerhalb des Einspritzbereichs mikrostrukturiert ist, |
| Fig. 22 | einen Vertikalschnitt durch ein weiteres Ausführungsbeispiel eines erfindungsgemäßen Mischkanals, |
| Fig. 23 | eine schematische Ansicht eines Vertikalschnitts durch die Längsmittelachse eines Ausführungsbeispiels eines erfindungsgemäßen Inhalators und |
| Fig. 24 | einen perspektivischen Ausschnitt des Inhalators nach Fig. 23. |

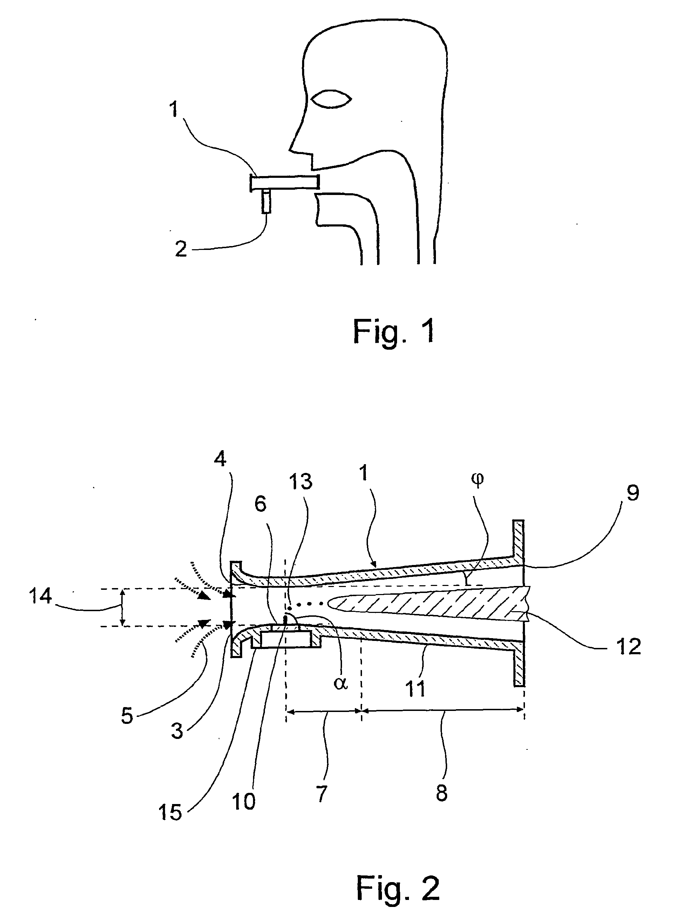

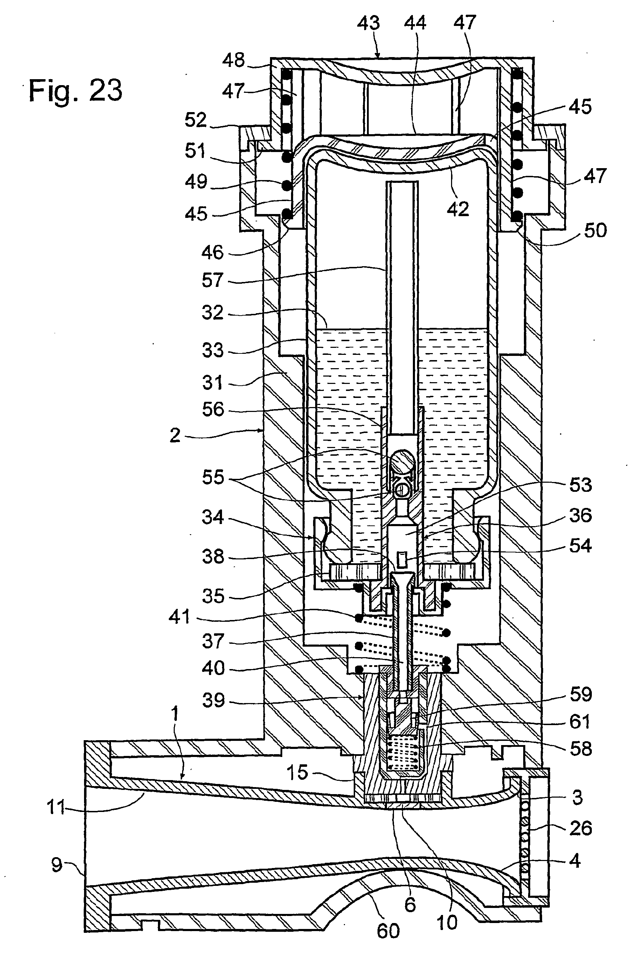

Der Inhalator nach Fig. 1 hat einen erfindungsgemäßen Mischkanal 1, der ein Mundstück bildet und von einem lungen- oder asthmakranken Patienten oder sonstigen Benutzer in den Mund eingeführt wird. Am Mischkanal 1 ist eine Dosiereinheit 2, hier mit einer herkömmlichen, manuell betätigbaren Pumpe, angeschlossen. Die Dosiereinheit 2 kann alternativ auch oben am Mischkanal 1 angebracht sein, wie es in Fig. 23 dargestellt ist. Durch die Dosiereinheit 2 wird ein flüssiges Medikament in den Mischkanal 1 eingespritzt, um es darin mit der Atemluft des Patienten zu einem Aerosol zu vermischen, das der Patient inhaliert.

Nach Fig. 2 saugt der Patient beim Inhalieren durch wenigstens eine Einlaßöffnung 3, wobei hier nur eine dargestellt ist, mit kreisrundem Durchtrittsquerschnitt und über einen sich trompetenförmig verengenden Einlaßbereich 4 Luft 5 in den Mischkanal 1 ein. Danach strömt die Luft weiter über einen als Düsenplatte ausgebildeten Einspritzbereich 6, einen Mischbereich 7, einen Endbereich 8 und eine Auslaßöffnung 9. In dem Einspritzbereich 6 ist wenigstens eine Düsenöffnung 10, hier ist wieder nur eine dargestellt, mit sehr kleiner Öffnungsweite von etwa 0,5 bis 5 $\mu$m ausgebildet. In dem hier gewählten Maßstab der Zeichnungen ist sie nicht sichtbar. Pro Düsenöffnung 10 tritt bei manueller Betätigung oder Auslösung des Druckhubs eines Kolbens einer Kolben-Zylinder-Einheit in der Dosiereinheit 2 ein anfänglich kontinuierlicher Strahl des flüssigen Medikaments unter einem Winkel $\alpha$ zu einer Tangente an den Einspritzbereich 6, hier senkrecht, aus dem Einspritzbereich 6 aus. Der zunächst kontinuierliche Strahl löst sich bereits in sehr geringem Abstand von der Innenseite der bei horizontaler Haltung des Mischkanals 1 unteren Kanalwand 11 in einen Strahl sehr feiner Tröpfchen 13 mit einem Durchmesser von etwa 1 bis 10 $\mu$m auf, wobei der Tröpfchendurchmesser vom Durchmesser der Düsenöffnung 10 abhängt. Die Tröpfchen 13 werden von der Luftströmung mitgerissen und mit dieser im Mischbereich 7 zu dem Aerosol 12 vermischt. Dabei ist eine Verengung 14 des Mischkanals 1 im Einspritzbereich 6 so klein ausgebildet, daß die Luft bei normaler Einatmungssaugkraft im Einspritzbereich 6 eine höhere Strömungsgeschwindigkeit als die in die Einlaßöffnung 3 eintretende Luft erhält. Dadurch wird mit hoher Sicherheit verhindert, daß die Tröpfchen 13 die dem Einspritzbereich 6 gegenüberliegende Kanalwand berühren und dort anhaften. Nach der Verengung 14 divergiert der Mischkanal 1 unter einem Winkel $\varphi$ relativ zu seiner Längsmittelachse (oder einer Parallelen zu dieser). Der Winkel $\varphi$ ist hierbei kleiner als 24° oder 14° gewählt, vorzugsweise kleiner als 10° und im Idealfall kleiner als 5°. Dadurch wird erreicht, daß die Luftströmung sich nicht von der Kanalwand löst und die Tröpfchen 13 nicht aufgrund von Luftwirbeln durch ein Ablösen des Luftstroms von der Kanalwand koagulieren und sich auch nicht an der Kanalwand niederschlagen. Sie bleiben daher monodispers (gleich groß) oder behalten einen monodispersen Charakter oder eine vorbestimmte Tröpfchengrößenverteilung bei. Gleichzeitig nimmt die Strömungsgeschwindigkeit wieder ab, so daß die Tröpfchen nicht mit zu hoher Geschwindigkeit in den Mundraum eintreten. Dadurch wird verhindert, daß sie dort an der Innenseite oder in der Kehle anhaften, ohne in die Lunge zu gelangen.

Ein den Einspritzbereich 6 umgebender Stutzen 15 dient zum Anschluß der Dosiereinheit 2.

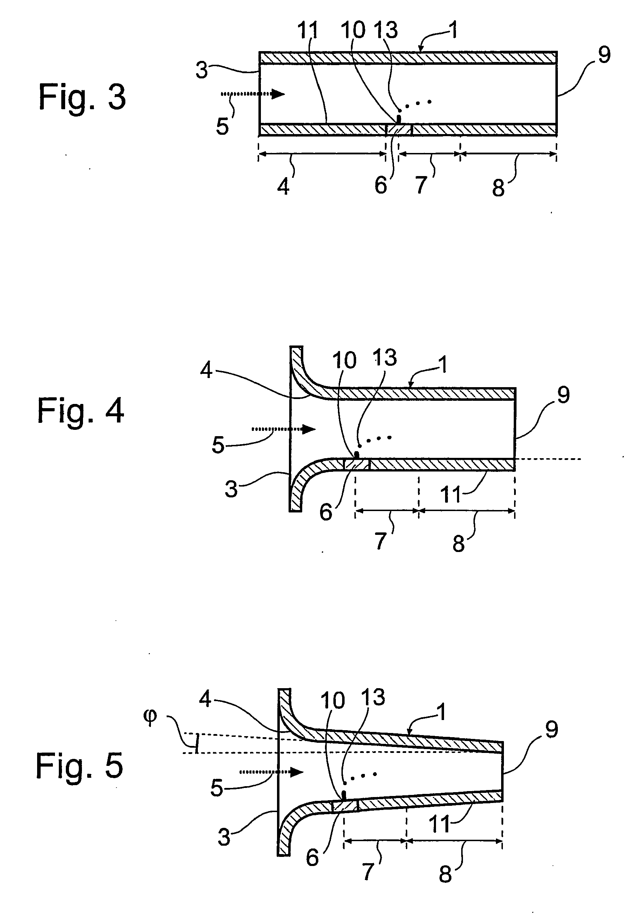

Bei dem Ausführungsbeispiel nach Fig. 3 ist der Mischkanal 1 über seine gesamte Länge geradlinig. Mithin ist hier der Winkel $\varphi$ gleich 0°. Sein Durchtrittsquerschnitt ist jedoch etwas größer als der Durchtrittsquerschnitt in der Verengung 14 des Mischkanals 1 nach Fig. 2 ausgebildet. Sein Einspritzbereich 6 liegt etwa in der Mitte seiner Länge. Die Lage und Länge seines Einlaßbereichs 4 und Endbereichs 7 sind entsprechend gegenüber denen des Mischkanals 1 nach Fig. 2 verschoben und verlängert bzw. verkürzt. Der Stutzen 15 kann entfallen und ist daher in Fig. 3 nicht dargestellt. Die Strömung der Luft bzw. des Aerosols bleibt weiterhin ablösungs- und wirbelfrei ab dem Einspritzbereich. Da die Luft aufgrund des größeren Durchtrittsquerschnitts etwas langsamer strömt, wird ebenfalls verhindert, daß sich die Tröpfchen 13 im Mundraum oder in der Kehle niederschlagen und dort haften bleiben.

Das Ausführungsbeispiel nach Fig. 4 unterscheidet sich von dem nach Fig. 2 nur dadurch, daß der Mischbereich 7 und der Endbereich 8 nicht divergieren, sondern über ihre gesamte Länge einen konstanten Durchtrittsquerschnitt haben, der etwas größer als der der Verengung 14 des Mischkanals 1 nach Fig. 2 ist. Die Wirkungsweise entspricht weitgehend der des Ausführungsbeispiels nach Fig. 3.

Das Ausführungsbeispiel nach Fig. 5 unterscheidet sich von dem nach Fig. 2 dadurch, daß der Durchtrittsquerschnitt des Mischkanals 1 über seine gesamte Länge konvergiert, und zwar zunächst bis etwa zur Mitte des Einlaßbereichs 4 trompetenförmig und danach geradlinig unter einem negativen Winkel φ gegenüber der Längsmittelachse des Mischkanals 1. Ferner liegt die engste Stelle des Mischkanals 1 an seinem Ende in der Auslaßöffnung 9. Der negative Winkel φ kann zwischen 0° und -77° liegen. Vorzugsweise liegt er zwischen 0° und -50°. Im dargestellten Ausführungsbeispiel beträgt er etwa -5°. Bis zu dem größten negativen Winkel φ von -77° löst sich die Luftströmung nicht von der Kanalwand ab, so daß eine Koagulation der Tröpfchen 13 durch Luftwirbel vermieden wird.

Die Verengung in der Auslaßöffnung 9 ist wieder etwas größer als die Verengung 14 nach Fig. 2, jedoch hinreichend groß, um eine Koagulation und ein Anhaften der Tröpfchen 13 im Mundraum und in der Kehle zu verhindern. Das Aerosol bekommt durch die Verengung eine vorbestimmte Richtung.

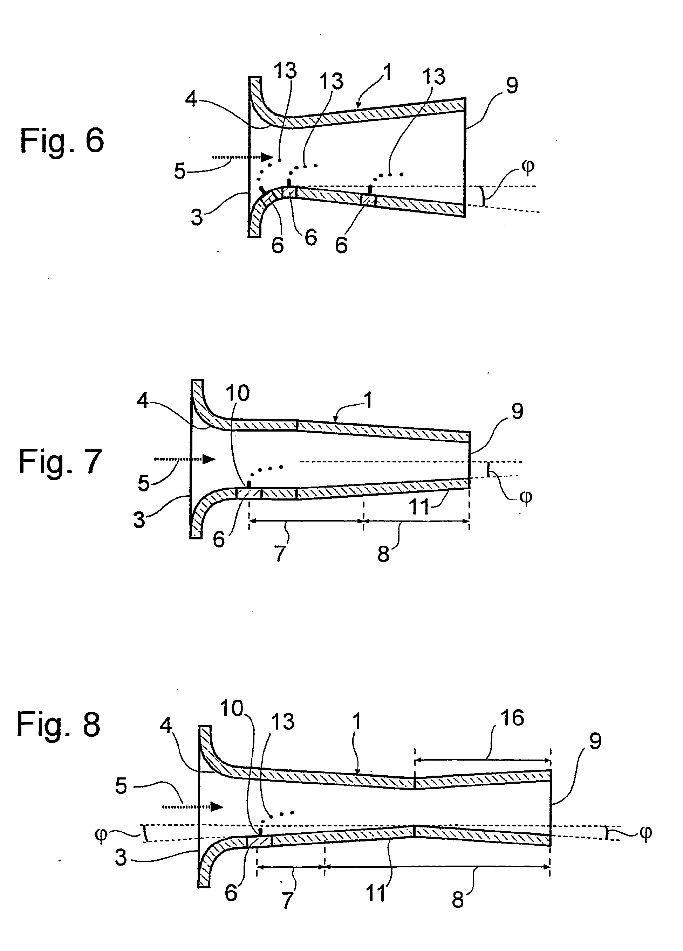

Fig. 6 stellt drei Ausführungsbeispiele dar, bei denen jeweils lediglich die Lage des Einspritzbereichs 6 gegenüber der Lage des Einspritzbereichs 6 nach Fig. 2 versetzt ist. Die Wirkungsweise bleibt hierbei im wesentlichen die gleiche wie die des Ausführungsbeispiels nach Fig. 2, nur daß die Austrittsrichtung des Strahls 13 bei dem Ausführungsbeispiel mit dem links dargestellten Einspritzbereich 6 im gekrümmten Einlaßbereich 4 schräg zur Strömungsrichtung der Luft 5 oder zur Längsmittelachse des Mischkanals 1 bzw. zur Einlaßöffnung 3 und bei dem Ausführungsbeispiel mit dem rechts dargestellten Einspritzbereich 6 zur Auslaßöffnung 9 hin geneigt ist. Bei dem Ausführungsbeispiel mit dem links dargestellten Einspritzbereich 6 wird der Strahl 13 schon frühzeitig in Tröpfchen aufgeteilt und bei dem Ausführungsbeispiel mit dem rechts dargestellten Einspritzbereich 6 besser von der Luftströmung eingehüllt, so daß die Tröpfchen mit höherer Sicherheit nicht gegen die Kanalwand fliegen.

Das Ausführungsbeispiel nach Fig. 7 unterscheidet sich von dem nach Fig. 2 dadurch, daß nach der trompetenförmigen Verringerung des Durchtrittsquerschnitts im Einlaßbereich 4 der Durchtrittsquerschnitt über den Einspritzbereich 6 hinweg bis in den Mischbereich 7 hinein konstant bleibt, um danach stetig unter dem Winkel φ von etwa -5° zur Längsmittelachse des Mischkanals 1 bis zur Auslaßöffnung 9 zu konvergieren. Die Wirkungsweise unterscheidet sich nicht wesentlich von der nach Fig. 5.

Das Ausführungsbeispiel nach Fig. 8 unterscheidet sich von dem nach Fig. 5 dadurch, daß der Endbereich 8 um einen unter dem Winkel φ von etwa 5° divergierenden Abschnitt 16 verlängert ist, so daß die Strömungsgeschwindigkeit des Aerosols im Abschnitt 16 wieder geringer wird und eine Koagulation und ein Haftenbleiben von Tröpfchen 13 im Mundraum mit höherer Sicherheit verhindert wird.

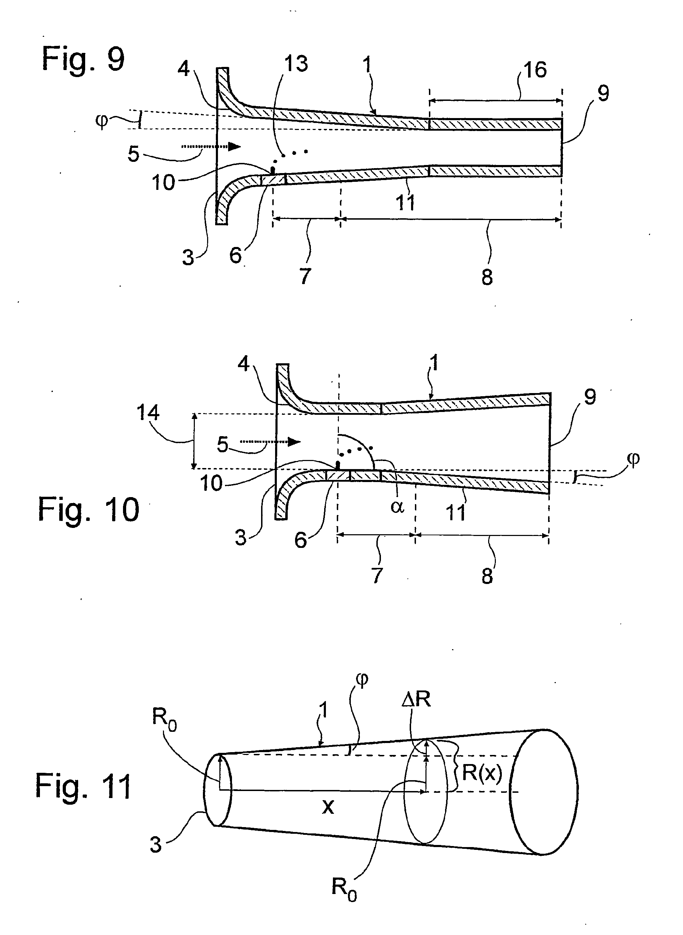

Bei dem Ausführungsbeispiel nach Fig. 9 hat der Abschnitt 16 gegenüber dem nach Fig. 8 einen konstanten Durchtrittsquerschnitt. Dadurch wird erreicht, daß das Aerosol mit hoher Geschwindigkeit weitergeleitet wird und aus der Auslaßöffnung 9 mit hoher Geschwindigkeit in einer vorbestimmten Richtung austritt, so daß es nicht an unerwünschten Stellen haften bleibt, sondern schnellstmöglich in Richtung der Lunge befördert wird.

Bei dem Ausführungsbeispiel nach Fig. 10 erstreckt sich die Verengung 14 mit konstantem Durchtrittsquerschnitt etwa gleich weit oder etwas weiter als bei Fig. 7 in den Mischbereich 7 hinein. Anschließend an die Verengung 14 geht der Durchtrittsquerschnitt nicht stetig, sondern in Form eines Knicks unter dem Winkel φ von etwa 5° in einen divergierenden Abschnitt über. Auch bei diesem Ausführungsbeispiel ist die Wirkungsweise im wesentlichen die gleiche wie die des Ausführungsbeispiels nach Fig. 2. Der Übergang von φ = 0° auf φ = 5° kann aber auch abgerundet sein. Dies trifft auch auf die Übergänge des Winkels φ bei den Ausführungsbeispielen nach den Fig. 7, 8 und 9 zu.

Der Winkel φ kann mithin im Bereich von -77° bis +24°, vorzugsweise im Bereich von -50° bis +14° oder +10°, noch spezieller zwischen 0° und +10° oder +5° oder zwischen -50° und 0°, liegen. Dabei ändert sich der Flächeninhalt $A(x)$ des Durchtrittsquerschnitts des Mischkanals 1 im Abstand x von der Einlaßöffnung 3, in Strömungsrichtung gesehen, gemäß Fig. 11, unabhängig von der Kontur des Durchtrittsquerschnitts, ebenso wie der eines hohlen Kegelstumpfes, bei dem der Flächeninhalt des Durchtrittsquerschnitts an der Einlaßöffnung 3 dem eines Kreises mit dem Radius $R_0$ und der halbe Öffnungswinkel dem Winkel φ entspricht. An der Stelle x ist dann der Radius

$$R(x) = R_0 + \Delta R \qquad\qquad (1)$$

wobei $\Delta R$ der Zunahme des Radius an der Stelle x entspricht. Mithin ist

$$\Delta R = x \tan \varphi \qquad\qquad (2)$$

und

$$A(x) = \pi(R_0 + \Delta R)^2 \qquad (3)$$

$$A(x) = \pi(R_0 + x\tan\varphi)^2 \qquad (4)$$

oder

$$A(x) = \pi(R_0^2 + 2R_0 x\tan\varphi + x^2\tan^2\varphi) \qquad (5)$$

Die Änderung des Flächeninhalts A(x) in Richtung x ist dann

$$dA(x)/dx = 2\pi R_0\tan\varphi + 2\pi x\tan^2\varphi \qquad (6)$$

$$= 2\pi R_0\tan\varphi(R_0 + x\tan\varphi) \qquad (7)$$

$$= 2\pi R(x)\tan\varphi \qquad (8)$$

An einer beliebigen Stelle x ist der Flächeninhalt A(x) des Durchtrittsquerschnitts mit

$$A(x) = \pi R(x)^2 \qquad (9)$$

und

$$R(x) = \sqrt{A(x)/\pi} \qquad (10)$$

Mithin

$$dA(x)/dx = 2\pi\sqrt{A(x)/\pi} \cdot \tan\varphi \qquad (11)$$

$$= 2\tan\varphi\sqrt{A(x)\cdot\pi} \qquad (12)$$

Das heißt, die Strömung im Mischkanal 1 ist an jeder Stelle x des Mischkanals 1 mit dem Flächeninhalt A(x) wirbelfrei und ablösungsfrei, wenn die Änderung der Fläche in Richtung x, d.h. dA(x)/dx an jeder Stelle x, kleiner als $2\tan\varphi\sqrt{A(x)\cdot\pi}$ und φ im Bereich von -77° bis +24° oder +14°, vorzugsweise im Bereich von -50° bis +5°, vorzugsweise weiter eingeengt zwischen -77° und 0° oder zwischen 0° und +10°, insbesondere zwischen -50° und 0° oder zwischen 0° und +5°, gehalten wird. Anders ausgedrückt: Die Änderung kann zwischen $-c_1\sqrt{A(x)}$ und 0 oder zwischen 0 und $c_2\sqrt{A(x)}$ liegen, wobei mit den angegebenen Maßzahlen für φ gilt, daß $C_1$ = 15,35 (bei -77°) oder 4,22 (bei -50°) und $C_2$ = 1,58 (bei 24°) oder 0,88 (bei 14°) oder 0,63 (bei 10°) oder 0,31 (bei 5°) ist.

Wenn der Durchtrittsquerschnitt des Mischkanals 1 nicht kreisförmig ist, läßt sich die Änderung des Flächeninhalts A(x) nach Gleichung (8) nicht berechnen. Für jeden Durchtrittsquerschnitt läßt sich aber ein entsprechender Radius R(x) für einen kreisförmigen Durchtrittsquerschnitt wie nach Gleichung (10) berechnen. Dieser Radius R(x) kann dann benutzt werden, um die Strömung im Mischkanal 1 an jeder Stelle x des Mischkanals 1 mit dem Flächeninhalt A(x) wirbelfrei und ablösungsfrei zu halten. Auch in diesem Fall gilt, daß die Änderung der Fläche in Richtung x, d.h. dA(x)/dx an jeder Stelle x, zwischen $-c_1\sqrt{A(x)}$ und 0 oder zwischen 0 und $c_2\sqrt{A(x)}$ liegt, wobei mit den angegebenen Maßzahlen für φ gilt, daß $c_1$ = 15,33 oder 4,22 und $c_2$ = 1,58; 0,88; 0,63 oder 0,31 ist.

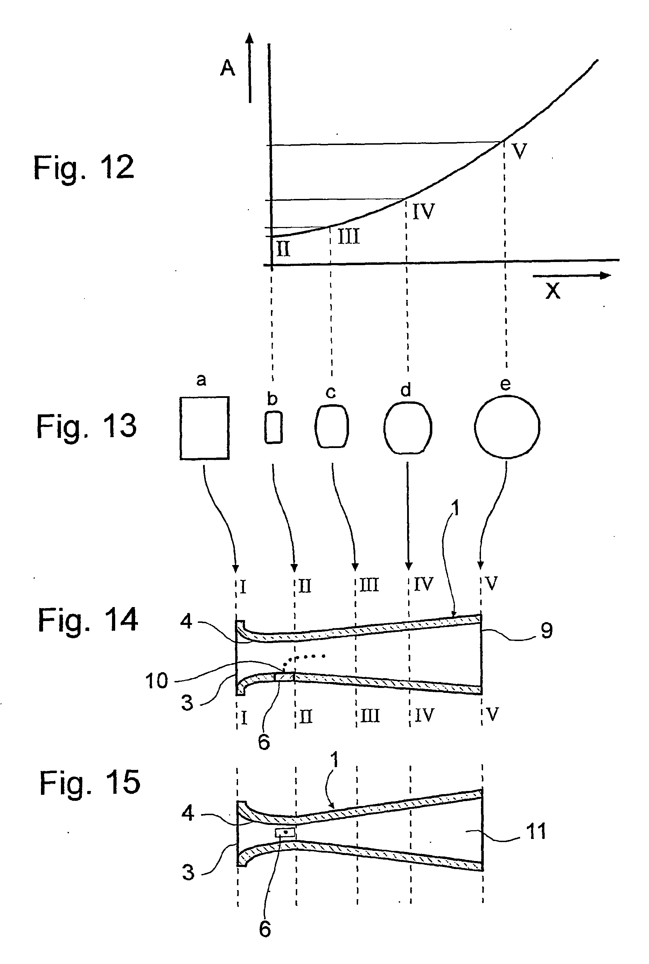

Fig. 12 veranschaulicht die Abhängigkeit des Flächeninhalts A des Durchtrittsquerschnitts von x gemäß Gleichung (5). Hierbei kann die Kontur oder Form der Fläche A oder des Durchtrittsquerschnitts sich so ändern, wie es in den Fig. 13a bis 13e dargestellt ist, d.h. der Durchtrittsquerschnitt des Mischkanals 1 kann in aufeinanderfolgenden Längsabschnitten von einer Rechteckform an der Einlaßöffnung 3 zu einer Rechteckform mit abgerundeten Ecken über den Einspritzbereich 6 stetig abnehmen und daran anschließend über Rechteckformen mit abgerundeten Ecken und nach außen gewölbten Seiten in eine Kreisform übergehen.

Ein entsprechender Mischkanal 1 ist in Fig. 14 im vertikalen Längsschnitt und in Fig. 15 im horizontalen Querschnitt, jeweils durch die Längsmittelachse, dargestellt, wobei die Querschnitte an den jeweiligen Stellen mit den Flächenformen gemäß den Fig. 13a bis 13e mit den römischen Zahlen I bis V und die zugehörigen Flächeninhalte der Querschnitte II bis V im Diagramm gemäß Fig. 12 mit den Zahlen II bis V bezeichnet sind.

Durch die Formgebung der Durchtrittsquerschnitte wird erreicht, daß der Durchtrittsquerschnitt im Einspritzbereich 6 optimal an die Einspritzung des monodispersen Aerosols angepaßt ist, nämlich rechteckig mit runden Ecken, rund an der Auslaßöffnung 9 zur Anpassung an den Mund des Patienten und in den Übergangsbereichen sich vergrößernd, um die Geschwindigkeit des Aerosols zu verringern.

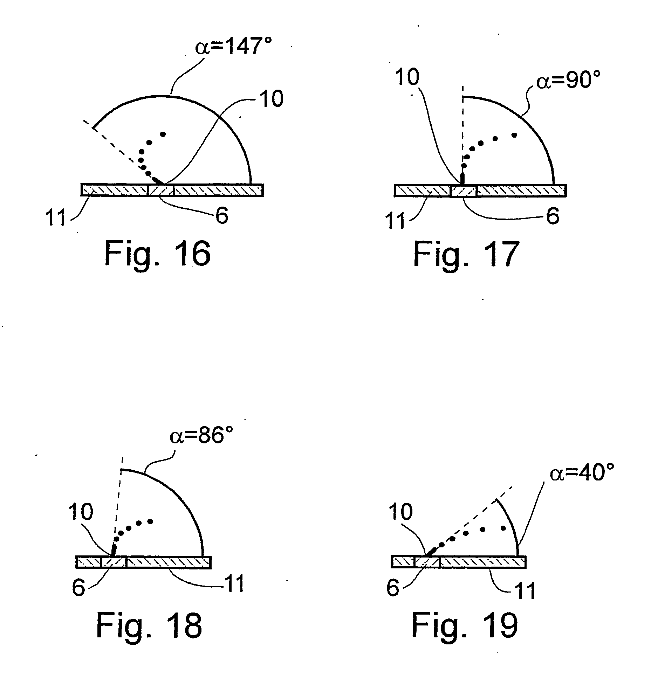

Die Fig. 16, 18 und 19 veranschaulichen anhand eines Ausschnitts der unteren Kanalwand 11, daß der Flüssigkeitsstrahl anfänglich nicht nur senkrecht zur Oberseite des Einspritzbereichs 6, wie es in Fig. 17 dargestellt ist, sondern auch unter einem anderen Winkel α zu einer Tangente am Einspritzbereich 6 austreten kann, insbesondere, wenn der Einspritzbereich 6 als Düsenplatte ausgebildet ist, in der die Richtung des zur Austrittsöffnung 10 führenden Kanals (oder bei mehreren Austrittsöffnungen 10 der Austrittsrichtung der zu den Austrittsöffnungen führenden Kanäle) einfacher ausgebildet werden kann. Dieser Winkel α kann im Bereich von 10° bis 170° liegen, und zwar unabhängig von dem Winkel φ.

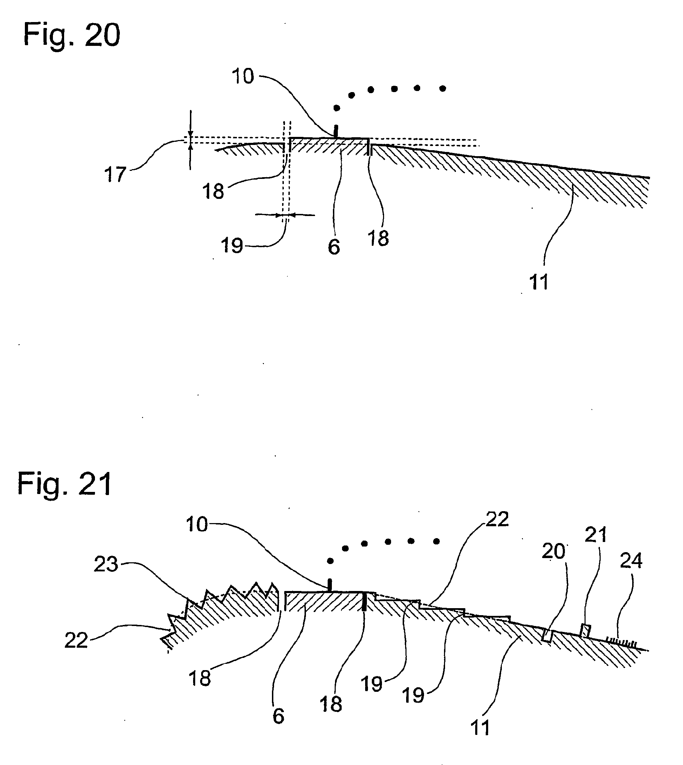

Wenn der Einspritzbereich 6 als Düsenplatte separat ausgebildet und nicht genau mit der Innenseite der Kanalwand 11 fluchtend in ein durchgehendes Loch oder eine Vertiefung der Kanalwand 11 eingesetzt wird, kann es sein, daß die Düsenplatte 6 gemäß dem vergrößerten Mischkanal-Ausschnitt nach Fig. 20 etwas über die Innenseite der Kanalwand 11 übersteht oder etwas zu tief in dem Loch oder in der Vertiefung liegt. Dies beeinträchtigt den ablösungsfreien Verlauf der Luftströmung weniger, wenn der Höhenunterschied 17 kleiner als 1 mm, vorzugsweise kleiner als 100 μm oder 20 μm ist. Desgleichen sollte ein seitlicher Spalt 18 zwischen der Wand des Loches oder der Vertiefung und der Düsenplatte 6 kleiner als 500 μm, vorzugsweise kleiner als 100 μm sein, um eine ablösungsfreie Luftströmung in dem Mischkanal beizubehalten.

Der vergrößerte Ausschnitt des Mischkanals nach Fig. 21 veranschaulicht eine weitere Abwandlung eines erfindungsgemäßen Mischkanals, bei dem die Innenseite der Kanalwand 11 mikrostrukturiert ist. So kann die Mikrostruktur in einem Verlauf 19 mit winzigen Treppen oder Rillen 20, Vorsprüngen 21, Sägezähnen 23 oder Mikrofasern 24 bestehen, die im Mittel den strichpunktiert dargestellten, gewünschten mittleren Oberflächenverlauf 22 ergeben. Dadurch wird der Luftströmungswiderstand der Innenseite des Mischkanals merklich verringert.

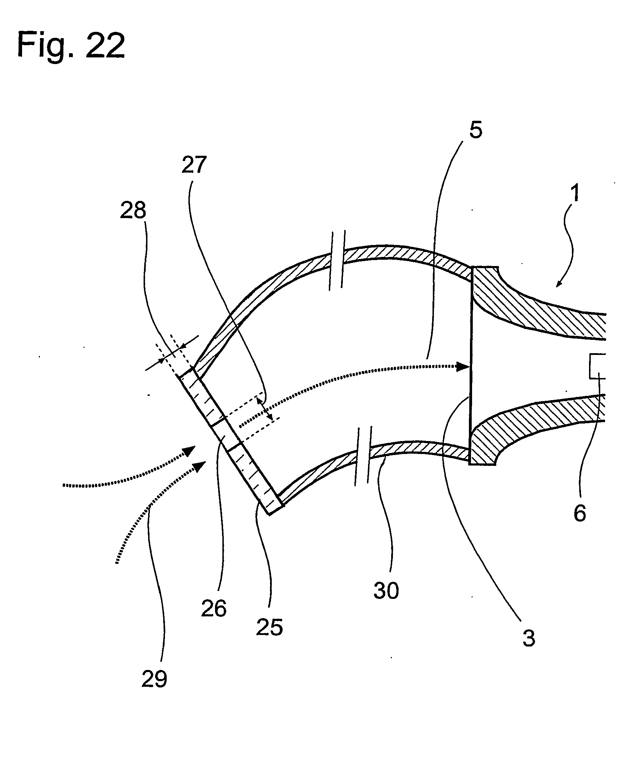

Eine vorteilhafte Weiterbildung kann nach Fig. 22 darin bestehen, daß an oder vor der Einlaßöffnung 3 ein Luftströmungswiderstand 25 in Form einer Lochplatte mit einem runden Loch 26 in der Mitte angeordnet ist. Der Durchmesser 27 des Loches beträgt 1 bis 12 mm, vorzugsweise 4 bis 8 mm. Die Länge 28 des Loches 26 ist so kurz wie möglich, vorzugsweise kleiner als der halbe Durchmesser 27. Vorzugsweise beträgt die Länge 28 0,1 bis 1 mm.

Um einen Eintritt von Luft 29 zwischen dem Luftströmungswiderstand 25 und der Einlaßöffnung 3 zu verhindern, wenn er nicht unmittelbar dicht an der Einlaßöffnung 3 ausgebildet oder angebracht ist, kann er durch ein gekrümmtes Zwischenrohr 30, z.B. einen Teil eines (nicht dargestellten) Gehäuses des Mischkanals, mit der Einlaßöffnung 3 verbunden sein. Wesentlich ist, daß die gesamte angesaugte Luft 29 durch das Loch 26 hindurchströmt.

Bei den angegebenen Maßen des Loches 26 nimmt die Strömungsgeschwindigkeit nichtlinear mit der Wurzel aus dem Druck, genauer: der Differenz der Drücke vor und hinter dem Loch, zu. Bei normalem Saugdruck mit nur etwa 2 bis 4 kPa ändert sich die Strömungsgeschwindigkeit der Luft im Loch 26 und Mischkanal nahezu linear, um dann mit zunehmendem Saugdruck weniger als proportional anzusteigen und sich schließlich mit weiter zunehmendem Saugdruck kaum noch zu ändern, was etwa einer Begrenzung der Strömungsgeschwindigkeit entspricht. Mit anderen Worten, bei geringer Saugkraft des Patienten wird die Strömungsgeschwindigkeit durch den Strömungswiderstand 25 kaum verringert. Bei maximaler Saugkraft ergibt sich dagegen eine kleinere Strömungsgeschwindigkeit als ohne den Strömungswiderstand 25.

Dies gibt dem Patienten ein besseres Gefühl beim Ansaugen der Luft, und die Strömungsgeschwindigkeit hängt weniger vom Patienten als von der Konstruktion ab. Er kann die Luft über längere Zeit gleichmäßig ansaugen.

Anstelle des runden Loches 26 kann auch ein eckiges, insbesondere quadratisches, dreieckiges oder schlitzförmiges, Loch vorgesehen sein. Auch mehrere Löcher in runder oder eckiger Form sind möglich, sofern ihr Strömungswiderstand insgesamt im wesentlichen mit dem des einen runden Loches 26 übereinstimmt. Ferner kann anstelle des gekrümmten Zwischenrohres 30 auch ein Teil des Gehäuses oder ein gerades Rohr vorgesehen sein.

Weitere Abwandlungen der Ausführungsbeispiele können beispielsweise darin bestehen, daß der Einspritzbereich 6 (bei horizontaler Haltung des Mischkanals 1) oberhalb der Längsmittelachse oder in der Seitenwand des Mischkanals 1 vorgesehen ist oder sich gegenüberliegende Einspritzbereiche 6 vorgesehen sind. Wenn der Einspritzbereich oben liegt, ist die Dosiereinheit 2 ebenfalls oben angeordnet. Bei mehreren Einspritzbereichen ist vorzugsweise nur eine Dosiereinheit 2 über Kanäle mit den Einspritzbereichen verbunden.

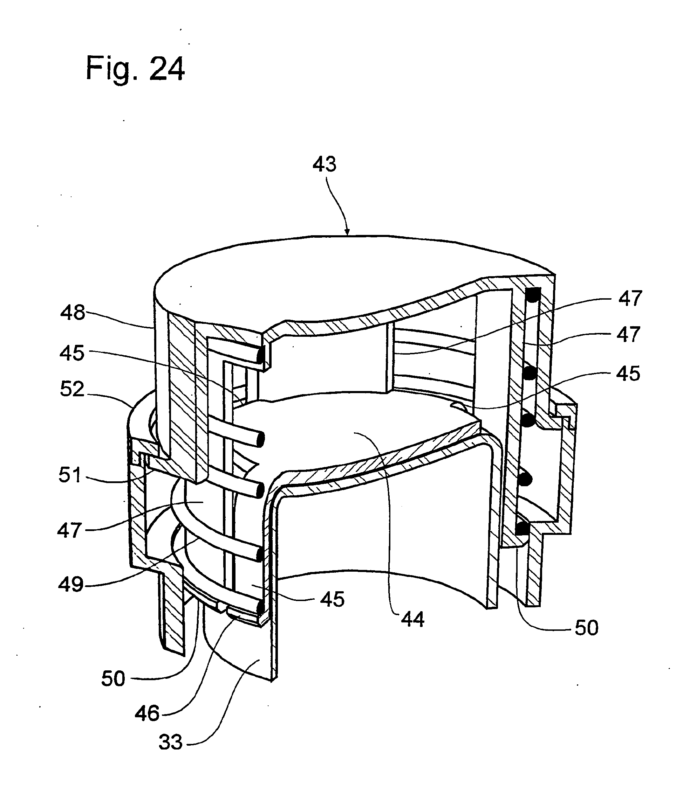

Fig. 23 stellt ein Ausführungsbeispiel eines vollständigen erfindungsgemäßen Inhalators im Axialschnitt in gegenüber der natürlichen Größe vergrößertem Maßstab und Fig. 24 den oberen Teil des Inhalators perspektivisch und aufgeschnitten in noch weiter vergrößertem Maßstab dar.

Der dargestellte Inhalator besteht aus dem Mischkanal 1 nach Fig. 2 und der Dosiereinheit 2 nach Fig. 1, d.h. die Dosiereinheit 2 ist in diesem Fall oben auf dem Mischkanal 1 angebracht. Abweichend von Fig. 2 hat der Mischkanal 1 jedoch einen Strömungswiderstand 25 gemäß

Fig. 22 in Form einer Lochplatte an der Einlaßöffnung 3. Allerdings hat diese Lochplatte anstelle nur eines Loches mehrere Löcher 26.

Die Dosiereinheit 2 hat ein Gehäuse 31, in dem der Mischkanal 1 und ein die Flüssigkeit 32 enthaltender Behälter 33 eingesetzt sind. Der Behälter 33 ist durch eine Verschlußeinrichtung 34 mit einer Dichtscheibe 35 dicht abgeschlossen. In der Verschlußeinrichtung 34 ist der Zylinder 36 einer Kolben-Zylinder-Anordnung dicht eingesetzt. Der Zylinder 36 ragt in den Behälter 33. Die Kolbenstange 37 des Kolbens 38 der Kolben-Zylinder-Anordnung ist durch die Verschlußeinrichtung 34 axial verschiebbar hindurchgeführt und mittels einer Einsatzanordnung 39 im Gehäuse 31 befestigt. Der Kolben 38 und die Kolbenstange 37 sind von einem bis zum Einspritzbereich 6 des Mischkanals 1 führenden Auslaßkanal 40 durchsetzt. Die Verschlußeinrichtung 34 stützt sich über eine weitere federnde Vorrichtung in Form einer Rückstellfeder 41 am Gehäuse 31 ab.

In einem Abstand vom Boden 42 des Behälters 33 ist ein Betätigungsteil 43 angeordnet, dessen Oberseite als Druckfläche zur manuellen Ausübung eines Drucks auf das Betätigungsteil 43 dient, um die Dosiereinheit 2 zu betätigen. Am Boden 42 des Behälters 33 liegt eine flache Schale 44 an, an deren Rand neben der Außenseite des Behälters 33 liegende Arme 45 (siehe auch Fig. 24) abgebogen sind, die an ihrem freien Ende jeweils einen Flansch 46 aufweisen. Zwischen den Armen 45 der Schale 44 sind von der Innenseite des Betätigungsteils 43 abstehende Arme 47 verschiebbar geführt. Zwischen den Armen 47 und einer zylindrischen Wand des Betätigungsteils 43 ist eine federnde Vorrichtung 49, hier in Form einer wendelförmigen Druckfeder (Schraubenfeder), angeordnet, die die Arme 45 und 47 umgibt und sich an der Unterseite des Bodens des Betätigungsteils 43 und auf den Flanschen 46 der Arme 45 sowie Flanschen 50 der Arme 47 abstützt. Das Betätigungsteil 43 ist mit einem umlaufenden Flansch 51 in dem Gehäuse 31 verschiebbar geführt und liegt unter dem Druck der federnden Vorrichtung 49 mit dem Flansch 51 an einem Ring 52 an, der am Gehäuse 31 befestigt ist und die Wand 48 des Betätigungsteils 43 mit Spiel (siehe Fig. 24) umgibt.

Der Druckraum 53 des Zylinders 36 steht über eine Öffnung 54 in seiner Wand, eine Ventilanordnung 55 in einem zylindrischen Fortsatz 56 des Zylinders 36 und ein in dem Fortsatz 56 befestigtes Tauchrohr 57 mit dem Innenraum des Behälters 33 in Verbindung. In der Lage der Dosiereinheit 2 nach Fig. 1, d.h. unterhalb des Mischkanals 1, ragt das freie Ende des Tauchrohrs 57 in die Flüssigkeit 32, und der Kolben 38 kann die Flüssigkeit, 32 über das Tauchrohr 57 und die Ventilanordnung 55 in den Druckraum 53 saugen. In der in Fig. 23 dargestellten Lage der Dosiereinheit dagegen, d.h. oben auf dem Mischkanal 1, kann die Flüssigkeit durch die Öffnung 54 in den Druckraum 53 des Zylinders 36 fließen und diesen sowie den Auslaßkanal 40 bis zu einem den Auslaßkanal 40 unter dem Druck einer Feder 58 absperrenden Ventilverschlußstück 59 füllen.

Bei Ausübung eines Drucks auf die Druckfläche des Betätigungsteils 43 mit dem Zeigefinger oder Daumen gegen den Druck des in eine Gehäuseaussparung 60 greifenden Daumens oder Zeigefingers wird das Betätigungsteil 43 gegen die Kraft der federnden Vorrichtung 49 bis zur Anlage der Flansche 51 seiner Arme 48 an einem inneren Absatz 62 des Gehäuses 31 gedrückt und die federnde Vorrichtung 49 gespannt, ohne daß sich zunächst der Behälter 33, einschließlich seiner Verschlußeinrichtung 34, ebenso weit wie das Betätigungsteil 43 relativ zum Gehäuse 31 verschiebt. Der Behälter 33 und die Verschlußeinrichtung 34 verschieben sich zunächst gegen die Kraft der Feder 41,

# EP 1 885 419 B1

die schwächer als die federnde Vorrichtung 49 ist, relativ zum gehäusefesten Kolben 38 nur so weit, bis der Kolben 38 die Öffnung 54 bzw. die Öffnung 54 den Kolben 38 überfahren hat. In diesem Augenblick wird die Flüssigkeit im Druckraum 53 unter Druck gesetzt und die Ventilanordnung 55 geschlossen, dagegen das Ventilverschlußstück 59 gegen die Kraft der Feder 58 in die Offenstellung verschoben, in der es eine Öffnung 61, die einen Teil des Auslaßkanals 40 bildet, überfahren hat. Das Betätigungsteil 43 wird jedoch weiterhin mit der Hand niedergedrückt gehalten, so daß nunmehr der Behälter 33 und die Verschlußeinrichtung 34 relativ zum feststehenden Kolben 38 durch die sich entspannende federnde Vorrichtung 49 verschoben werden, jedoch nur so langsam und in dem Maße, wie die Flüssigkeit durch die sehr enge und daher wie ein Drosselventil wirkende Düsenöffnung 10 im Einspritzbereich 6 in den Mischkanal 1 austreten kann. Infolgedessen sind der Druck und die Geschwindigkeit, unter dem bzw. mit der die Flüssigkeit in den Mischkanal 1 gespritzt wird, weitgehend unabhängig von der Kraft und Geschwindigkeit, mit denen das Betätigungsteil 43 manuell betätigt wird. Vielmehr hängen der Druck auf die Flüssigkeit und ihre Geschwindigkeit beim Einspritzen sowie die Dauer der Einspritzung in den Mischkanal 1 im wesentlichen nur von der Kraft der federnden Vorrichtung 49 ab, die an die zur Einhaltung einer gewünschten Inhalationsdauer von etwa 0,5 bis 10 Sekunden und einer entsprechenden Strömungsgeschwindigkeit des Strahls 13 weitgehend angepaßt werden kann. Der Benutzer des Inhalators hat somit weniger Einfluß auf den Betätigungsdruck, was die Handhabung des Inhalators erleichtert.

Nachdem der Druckraum 53 des Zylinders 36 entleert worden ist, wird der manuelle Druck auf das Betätigungsteil 43, weggenommen, so daß die federnde Vorrichtung 49 das Betätigungsteil 43 sowie die Feder 41 die Verschlußeinrichtung 34 samt Behälter 33 wieder in die dargestellte Ausgangslage zur Ausführung der nächsten Betätigung zurückstellen. Während der Rückstellung drückt die Feder 58 das Ventilverschlußstück 59 wieder in die dargestellte Schließlage, um ein Rücksaugen der Flüssigkeit 2 in den Zylinder 36 zu verhindern.

Statt der dargestellten federnden Vorrichtung 49 kann auch eine andere oder anders angeordnete federnde Vorrichtung vorgesehen sein. So kann der Behälter 33 selbst als federnde Vorrichtung ausgebildet sein, beispielsweise so, daß er vollständig oder nur ein Teil seiner Wand federnd ausgebildet ist und/oder der Behälter 33 sich mit seinem in Fig. 23 unten liegenden Öffnungsrand auf einem elastischen oder elastisch biegsamen Teil in der Verschlußeinrichtung 34 abstützt. In diesem Fall kann der Boden des Behälters 33 das Betätigungsteil bilden, so daß das Betätigungsteil 43, die Schale 44 und die federnde Vorrichtung 49 entfallen. Eine weitere Alternative kann darin bestehen, daß die federnde Vorrichtung 49 unmittelbar zwischen den Böden des Betätigungsteils 43 und der Schale 44 angeordnet ist. Oder die federnde Vorrichtung 49 kann unmittelbar zwischen dem Boden des Betätigungsteils 43 und dem Boden 42 des Behälters 33 angeordnet sein, so daß die Schale 44 entfällt. Nicht zuletzt ist es möglich, anstelle der federnden Vorrichtung 49 eine Druckfeder zwischen der Innenseite des Bodens 42 des Behälters 33 und einer am Tauchrohr 57 ausgebildeten Stützfläche anzuordnen, so daß wiederum das Betätigungsteil 43, die Schale 44 und die federnde Vorrichtung 49 entfallen können und der Boden 42 des Behälters 33 das Betätigungsteil bildet.

Als Dosiereinheit 2 sind neben einer herkömmlichen Pumpe auch andere Vorrichtungen geeignet, die das gewünschte Flüssigkeitsvolumen mit dem erforderlichen Druck erzeugen können. Bevorzugt wird eine Patronen-Dosiereinheit mit einer Patrone und einer Feder zur selbsttätigen Dosierung der Flüssigkeit (des gewünschten Medikaments oder eines flüssigen Mittels zur Behandlung des Mundes, Rachens oder der Lunge). Hierbei sind die Dosiereinheit und der Einspritzbereich durch einen Kanal miteinander verbunden. Der Kanal ist vorzugsweise ein Rohr oder Schlauch oder im Gehäuse des Mischkanals ausgebildet. Bei Anwendung einer Patronen-Dosiereinheit kann der Mischkanal in deren Verlängerung ausgebildet sein, so daß beide einen langgestreckten Halter in sogenannter Penform bilden.

## Patentansprüche

1.  Inhalator mit einem Mischkanal (1) zum Erzeugen eines zu inhalierenden Aerosols, wobei der Mischkanal aufweist: eine in den Mund einer Person einzuführende Auslassöffnung (9) an seinem einen Ende zum Inhalieren des erzeugten Aerosols; wenigstens eine Einlassöffnung (3) an seinem anderen Ende zum Einsaugen von Luft in den Mischkanal (1) und wenigstens einen zwischen der Einlassöffnung (3) und der Auslassöffnung (9) liegenden, einen Teil der Kanalwand bildenden Einspritzbereich (6), wobei der Einspritzbereich (6) wenigstens eine Düsenöffnung (10) zum Zuführen einer Flüssigkeit, insbesondere eines flüssigen Medikaments aufweist, wobei die Innenseite des Einspritzbereichs (6) zumindest mit dem ihr einlassseitig benachbarten Teil des mittleren Oberflächenverlaufs (22) der Innenseite der Kanalwand (11) weitgehend fluchtet, wobei ein etwaiger Höhenunterschied (17) zwischen der Innenseite des Einspritzbereichs (6) und dem ihr benachbarten Teil des mittleren Oberflächenverlaufs (22) der Innenseite der Kanalwand (11) kleiner als 1 mm ist oder höchstens 100 $\mu$m oder höchstens 20 $\mu$m beträgt, **dadurch gekennzeichnet, dass** die Kanalwand ansonsten fluidzuführungsfrei ausgebildet ist und das Zuführen der Flüssigkeit durch Einspritzen der Flüssigkeit in Form eines Strahls pro Düsenöffnung (10) aus dispersen Tröpfchen (13) in den Mischkanal erfolgt, um die Tröpfchen (13) durch den eingesaugten Luftstrom mitzureißen und in einem Endbereich (8) zwischen Einspritzbereich (6) und Auslassöffnung (9) getrennt zu halten, nachdem sich die Tröpfchen (13) mit dem Luftstrom zu dem Aerosol vermischt haben.

**2.** Inhalator nach Anspruch 1, **dadurch gekennzeichnet, daß** sich der Flächeninhalt A(x) des Durchtrittsquerschnitts des Mischkanals (1), unabhängig von der Kontur des Durchtrittsquerschnitts, in Luftströmungsrichtung x so ändert, daß die Änderung dA(x)/dx an jeder Stelle x zwischen $-c_1\sqrt{A(x)}$ und 0 oder zwischen $c_2\sqrt{A(x)}$ und 0 liegt und $c_1$ = 15,35 oder 4,22 und $c_2$ = 1,58 oder 0,88 oder 0,31 ist.

**3.** Inhalator nach Anspruch 1 oder 2, **dadurch gekennzeichnet, daß** der Mischkanal (1) über den Einspritzbereich (6) hinweg linear oder nichtlinear divergiert und/oder konvergiert oder parallel zur Längsmittelachse verläuft.

**4.** Inhalator nach einem der Ansprüche 1 bis 3, **dadurch gekennzeichnet, daß** der Mischkanal (1) in einem sich über den Einspritzbereich (6) hinweg erstreckenden Abschnitt konvergiert und stromunterhalb des konvergierenden Abschnitts divergiert.

**5.** Inhalator nach einem der Ansprüche 1 bis 4, **dadurch gekennzeichnet, daß** der Mischkanal (1) von der Einlaßöffnung (3) bis zum Einspritzbereich (6) trompetenartig konvergiert.

**6.** Inhalator nach einem der Ansprüche 1 bis 5, **dadurch gekennzeichnet, daß** die Axialschnittkontur der Innenseite des trompetenartig konvergierenden Abschnitts der Kanalwand zumindest oberhalb und unterhalb der Längsmittelachse dem Verlauf einer Parabel dritten Grades entspricht.

**7.** Inhalator nach einem der Ansprüche 1 bis 6, **dadurch gekennzeichnet, daß** der Durchtrittsquerschnitt des Mischkanals (1) in aufeinanderfolgenden Längsabschnitten von einer Rechteckform an der Einlaßöffnung (3) zu einer Rechteckform mit abgerundeten Ecken über den Einspritzbereich (6) hinweg stetig abnimmt und daran anschließend über Rechteckformen mit abgerundeten Ecken und nach außen gewölbten Seiten in eine Kreisform übergeht.

**8.** Inhalator nach einem der Ansprüche 1 bis 7, **dadurch gekennzeichnet, daß** der Strahl unter einem Winkel $\alpha$ zu einer Tangente an den Einspritzbereich zwischen 10° und 170°, vorzugsweise zwischen 10° und 90° oder zwischen 90° und 170°, aus dem Einspritzbereich (6) austritt, und der Strahl anfänglich bei $\alpha < 90°$ zur Auslaßöffnung (9) und bei $\alpha > 90°$ zur Einlaßöffnung (3) hin geneigt ist.

**9.** Inhalator nach einem der Ansprüche 1 bis 8, **dadurch gekennzeichnet, daß** die Länge eines sich an den Einspritzbereich (6) in Axialrichtung des Mischkanals (1) anschließenden Mischbereichs (7) 1 bis 200 mm oder 1 bis 50 mm beträgt.

**10.** Inhalator nach Anspruch 9, **dadurch gekennzeichnet, daß** die Länge eines sich an den Mischbereich (7) anschließenden Endbereichs (8) das 2- bis 3-fache der Länge des Mischbereichs (7) beträgt.

**11.** Inhalator nach einem der Ansprüche 1 bis 10, **dadurch gekennzeichnet, daß** der Flächeninhalt des Durchtrittsquerschnitts des Mischkanals (1) am Ende eines sich bis zum Einspritzbereich (6) erstreckenden Einlaßbereichs (4) 1 bis 1000 mm$^2$ oder 5 bis 200 mm$^2$, vorzugsweise 10 bis 20 mm$^2$, beträgt.

**12.** Inhalator nach einem der Ansprüche 1 bis 11, **dadurch gekennzeichnet, daß** der Einspritzbereich (6) durch eine Düsenplatte gebildet ist.

**13.** Inhalator nach einem der Ansprüche 1 bis 12, **dadurch gekennzeichnet, daß** der Mischkanal (1) Kunststoff mit oder ohne einen antibakteriellen oder elektrisch leitfähigen Zusatz oder Belag aufweist.

**14.** Inhalator nach einem der Ansprüche 1 bis 13, **dadurch gekennzeichnet, daß** die Oberfläche der Innenseite der Kanalwand (11) mindestens teilweise mikrostrukturiert ist.

**15.** Inhalator nach einem der Ansprüche 1 bis 14, **dadurch gekennzeichnet, daß** an oder vor der Einlaßöffnung (3) ein Luftströmungswiderstand (25) angeordnet ist.

**16.** Inhalator nach Anspruch 15, **dadurch gekennzeichnet, daß** der Luftströmungswiderstand (25) eine Lochplatte mit wenigstens einem Loch ist.

**17.** Inhalator nach einem der Ansprüche 1 bis 16, **dadurch gekennzeichnet, daß** an dem Mischkanal (1) eine manuell betätigbare Dosiereinheit (2) für die Flüssigkeit mit einem Volumenbereich von 1 bis 300 Mikroliter vorgesehen ist.

18. Inhalator nach Anspruch 17, **dadurch gekennzeichnet, daß** die Dosiereinheit (2) eine Kolben-Zylinder-Anordnung (36, 38) zur Ausübung eines Drucks auf die in den Mischkanal (1) einzuspritzende Flüssigkeit (32) und ein durch einen manuellen Druck bewegbares Betätigungsteil (43) aufweist, dessen Betätigungsweg über eine federnde Vorrichtung (49) in eine Relativbewegung zwischen dem Kolben (38) und dem Zylinder (36) der Kolben-Zylinder-Anordnung umwandelbar ist.

19. Inhalator nach Anspruch 18, **dadurch gekennzeichnet, daß** der Zylinder (36) in einer Verschlußeinrichtung (34) eines die Flüssigkeit (32) enthaltenden Behälters (33) dicht eingesetzt ist und in den Behälter (33) ragt, daß eine Kolbenstange (37) des Kolbens (38) durch die Verschlußeinrichtung (34) hindurchgeführt ist, daß der Kolben (38) und die Kolbenstange (37) von einem bis zu dem Einspritzbereich (6) führenden Auslaßkanal (40) durchsetzt sind, daß die Kolbenstange (37) in einem die Verschlußeinrichtung (34) verschiebbar aufnehmenden Gehäuse (31) befestigt ist, daß der Betätigungsweg des Betätigungsteils (43) über die federnde Vorrichtung (49) gegen die Kraft einer weiteren federnden Vorrichtung (41) auf die Verschlußeinrichtung (34) übertragbar ist, so daß die Verschlußeinrichtung (34) relativ zum Kolben (38) verschoben wird, daß der Druckraum (53) des Zylinders (36) im unbetätigten Zustand des Betätigungsteils (43) über wenigstens eine Öffnung (54) in der Wand des Zylinders (36) und über eine Ventilanordnung (55) mit dem Innenraum des Behälters (33) in Verbindung steht und daß die Öffnung (en) (54) des Zylinders (36) und die Ventilanordnung (55), nach Überfahren der Öffnung (en) (54) des Zylinders (36) durch den Kolben (38), während der Betätigung des Betätigungsteils (43) gesperrt sind.

**Claims**

1. An inhaler with a mixing channel (1) for producing an aerosol to be inhaled, where the mixing channel has: an outlet (9) at one end that can be inserted in the mouth of a person in order to inhale the aerosol that is produced; at least one inlet (3) at its other end for drawing air into the mixing channel (1) and at least one injection zone (6) that lies between the inlet (3) and the outlet (9) and forms part of the channel wall, where the injection zone (6) has at least one nozzle orifice (10) for supplying a liquid, especially a liquid drug, wherein the inner surface of the injection zone (6) is largely flush at least with that portion of the mean surface curvature (22) of the inner surface of the channel wall (11) that is adjacent to it on the inlet end, where a possible height difference (17) between the inner surface of the injection zone (6) and that portion of the mean surface curvature (22) of the inner surface of the channel wall (11) that is adjacent to it is less than 1 mm or is at most 100 $\mu$m or at most 20 $\mu$m, **characterized in, that** the channel wall otherwise is formed free of fluid supplies, and that the supply of liquid is performed by injecting the liquid into the mixing channel in the form of one jet of dispersed droplets (13) per nozzle orifice (10) so as to entrain the droplets (13) with the stream of intake air and keep them separated in an end zone (8) between the injection zone (6) and the outlet opening (9) after the droplets (13) have been mixed with the stream of air to form the aerosol.

2. An inhaler in accordance with Claim 1, **characterized in that** the area A(x) of the passage cross section of the mixing channel (1) varies in the air flow direction x, independently of the contour of the passage cross section, in such a way that the change dA(x)/dx at each point x is between $-c_1\sqrt{A(x)}$ and 0 or between $c_2\sqrt{A(x)}$ and 0, where $c_1$ = 15.35 or 4.22, and $c_2$ = 1.58 or 0.88 or 0.31.

3. An inhaler in accordance with Claim 1 or Claim 2, **characterized in, that** the mixing channel (1) diverges and/or converges linearly or nonlinearly beyond the injection zone (6) or runs parallel to the longitudinal center axis.

4. An inhaler in accordance with any of Claims 1 to 3, **characterized in, that** the mixing channel (1) converges in a section that extends beyond the injection zone (6) and then diverges downstream of the convergent section.

5. An inhaler in accordance with any of Claims 1 to 4, **characterized in, that** the mixing channel (1) converges like a trumpet from the inlet (3) to the injection zone (6).

6. An inhaler in accordance with any of Claims 1 to 5, **characterized in, that** the axial section contour of the inner surface of the section of the channel wall that converges like a trumpet corresponds to the curve of a parabola of third degree, at least above and below the longitudinal center axis.

7. An inhaler in accordance with any of Claims 1 to 6, **characterized in, that** the passage cross section of the mixing channel (1) continuously decreases in successive longitudinal sections from a rectangular shape at the inlet (3) to a rectangular shape with rounded corners across the injection zone (6), and it then makes a transition from rectangular shapes with rounded corners and outwardly arched sides to a circular shape.

8. An inhaler in accordance with any of Claims 1 to 7, **characterized in, that** the jet emerges from the injection zone (6) at an angle $\alpha$ to a tangent to the injection zone of 10-170°, and preferably 10-90° or 90-170°, and that the jet has an initial inclination to the outlet (9) of $\alpha < 90°$ and an initial inclination to the inlet (3) of $\alpha > 90°$.

9. An inhaler in accordance with any of Claims 1 to 8, **characterized in, that** the length of a mixing zone (7) that follows the injection zone (6) in the axial direction of the mixing channel (1) is 1-200 mm or 1-50 mm.

10. An inhaler in accordance with Claim 9, **characterized in, that** the length of an end zone (8) that follows the mixing zone (7) is 2-3 times the length of the mixing zone (7).

11. An inhaler in accordance with any of Claims 1 to 10, **characterized in, that** the area of the passage cross section of the mixing channel (1) at the end of an inlet zone (4) that extends to the injection zone (6) is 1 to 1,000 mm$^2$ or 5 to 200 mm$^2$, and preferably 10 to 20 mm$^2$.

12. An inhaler in accordance with any of Claims 1 to 11, **characterized in, that** the injection zone (6) is formed by a nozzle plate.

13. An inhaler in accordance with any of Claims 1 to 12, **characterized in, that** the mixing channel (1) contains plastic with or without an antibacterial or electrically conductive additive or coating.

14. An inhaler in accordance with any of Claims 1 to 13, **characterized in, that** the surface of the inside of the channel wall (11) is at least partially micro textured.

15. An inhaler in accordance with any of Claims 1 to 14, **characterized in, that** an air flow resistor (25) is installed on or in front of the inlet (3).

16. An inhaler in accordance with Claim 15, **characterized in, that** the air flow resistor (25) is a perforated plate with at least one hole.

17. An inhaler in accordance with any of Claims 1 to 16, **characterized in, that** a manually operated metering unit (2) for the liquid is provided on the mixing channel (1) and that it has a volume metering range of 1 to 300 microliters.

18. An inhaler in accordance with any of Claims 1 to 16, **characterized in, that** the metering unit (2) has a piston-cylinder system (36, 38) for applying pressure to the liquid (32) to be injected into the mixing channel (1) and an actuator (43) that can be moved by manual pressure, whose actuation distance can be converted by a spring mechanism (49) to relative movement between the piston (38) and the cylinder (36) of the piston-cylinder system.

19. An inhaler in accordance with Claim 18, **characterized in, that** the cylinder (36) is tightly mounted in a closure device (34) of a reservoir (33) that contains the liquid (32) and extends into the reservoir (33), that a piston rod (37) of the piston (38) is passed through the closure device (34), that the piston (38) and the piston rod (37) are interstratified by an outlet channel (40) that extends to the injection zone (6), that the piston rod (37) is mounted in a housing (31) that displaceably holds the closure device (34), that the actuation distance of the actuator (43) can be transferred to the closure device (34) by the spring mechanism (49) against the force of another spring mechanism (41), so that the closure device (34) is moved relative to the piston (38), that the pressure chamber (53) of the cylinder (36) in the unactuated state of the actuator (43) is connected with the interior of the reservoir by at least one hole (54) in the wall of the cylinder (36) and by a valve system (55) and that the hole(s) (54) in the wall of the cylinder (36) and the valve system (55) are blocked during the operation of the actuator (43) after the piston (38) has travelled beyond the hole(s) (54) in the wall of the cylinder (36).

**Revendications**

1. Inhalateur doté d'un canal mélangeur (1) destiné à la production d'un aérosol à inhaler, dans lequel le canal mélangeur présente: une ouverture de sortie (9) à introduire dans la bouche d'une personne à sa première extrémité pour l'inhalation de l'aérosol produit; au moins une ouverture d'entrée (3) à son autre extrémité pour l'aspiration d'air dans le canal mélangeur (1), et au moins une zone d'injection (6) formant une partie de la paroi du canal et située entre l'ouverture d'entrée (3) et l'ouverture de sortie (9), dans lequel la zone d'injection (6) présente au moins une ouverture d'injecteur (10) pour l'introduction d'un liquide, en particulier d'un médicament liquide, dans lequel le côté intérieur de la zone d'injection (6) est largement aligné au moins avec la partie proche de celui-ci côté entrée du profil de surface moyen (22) du côté intérieur de la paroi du canal (11), dans lequel une éventuelle différence de hauteur (17) entre le côté intérieur de la zone d'injection (6) et la partie proche de celui-ci du profil de surface moyen (22) du côté intérieur de la paroi du canal (11) est inférieure à 1 mm ou vaut au maximum 100 $\mu$m ou au maximum 20 $\mu$m, **caractérisé en ce que** la paroi du canal est par ailleurs libre de guidage de fluide et l'introduction du liquide par injection du liquide sous la forme d'un jet de gouttelettes dispersées (13) par ouverture de tuyère (10) dans le canal mélangeur est effectuée en vue d'entraîner les gouttelettes (13) au moyen du courant d'air aspiré et de les maintenir séparées dans une zone d'extrémité (8) entre la zone d'injection (6) et l'ouverture de sortie (9), après que les gouttelettes (13) se soient mélangées avec le courant d'air pour former l'aérosol.

2.  Inhalateur selon la revendication 1, **caractérisé en ce que** la superficie A(x) de la section transversale de passage du canal mélangeur (1), indépendamment du contour de la section transversale de passage, varie dans la direction d'écoulement de l'air x de telle manière que la variation dA(x)/dx en chaque point x soit comprise entre $-c_1\sqrt{A(x)}$

    et 0 ou entre $c_2\sqrt{A(x)}$ et 0 et $c_1$ = 15,35 ou 4,22 et $c_2$ = 1,58 ou 0,88 ou 0,31.

3.  Inhalateur selon la revendication 1 ou 2, **caractérisé en ce que** le canal mélangeur (1) diverge et/ou converge de façon linéaire ou non linéaire au-delà de la zone d'injection (6) ou il s'étend parallèlement à l'axe central longitudinal.

4.  Inhalateur selon l'une quelconque des revendications 1 à 3, **caractérisé en ce que** le canal mélangeur (1) converge sur une partie s'étendant au-delà de la zone d'injection (6) et diverge en aval de la partie convergente.

5.  Inhalateur selon l'une quelconque des revendications 1 à 4, **caractérisé en ce que** le canal mélangeur (1) converge en forme de trompette depuis l'ouverture d'entrée (3) jusqu'à la zone d'injection (6).

6.  Inhalateur selon l'une quelconque des revendications 1 à 5, **caractérisé en ce que** le contour en coupe axiale du côté intérieur de la partie de la paroi du canal convergente en forme de trompette correspond au tracé d'une parabole du troisième degré au moins au-dessus et en dessous de l'axe central longitudinal.

7.  Inhalateur selon l'une quelconque des revendications 1 à 6, **caractérisé en ce que** la section transversale de passage du canal mélangeur (1) diminue en continu, dans des parties longitudinales successives, d'une forme rectangulaire à l'ouverture d'entrée (3) à une forme rectangulaire avec des coins arrondis au-delà de la zone d'injection (6) et acquiert ensuite une forme circulaire par des formes rectangulaires avec des arrondis et des côtés bombés vers l'extérieur.

8.  Inhalateur selon l'une quelconque des revendications 1 à 7, **caractérisé en ce que** le jet sort de la zone d'injection (6) sous un angle $\alpha$ par rapport à une tangente à la zone d'injection compris entre 10° et 170°, de préférence entre 10° et 90° ou entre 90° et 170°, et le jet est initialement incliné de $\alpha < 90°$ vers l'ouverture de sortie (9) et de $\alpha > 90°$ vers l'ouverture d'entrée (3).

9.  Inhalateur selon l'une quelconque des revendications 1 à 8, **caractérisé en ce que** la longueur d'une zone de mélange (7) se raccordant à la zone d'injection (6) dans la direction axiale du canal mélangeur (1) vaut 1 à 200 mm ou 1 à 50 mm.

10. Inhalateur selon la revendication 9, **caractérisé en ce que** la longueur d'une zone finale (8) se raccordant à la zone de mélange (7) vaut 2 à 3 fois la longueur de la zone de mélange (7).

11. Inhalateur selon l'une quelconque des revendications 1 à 10, **caractérisé en ce que** la superficie de la section transversale de passage du canal mélangeur (1) à l'extrémité d'une zone d'entrée (4) s'étendant jusqu'à la zone d'injection (6) vaut 1 à 1000 mm$^2$ ou 5 à 200 mm$^2$, de préférence 10 à 20 mm$^2$.

**12.** Inhalateur selon l'une quelconque des revendications 1 à 11, **caractérisé en ce que** la zone d'injection (6) est formée par une plaque à injecteur.

**13.** Inhalateur selon l'une quelconque des revendications 1 à 12, **caractérisé en ce que** le canal mélangeur (1) présente une matière plastique avec ou sans un additif ou un revêtement antibactérien ou électriquement conducteur.

**14.** Inhalateur selon l'une quelconque des revendications 1 à 13, **caractérisé en ce que** la surface du côté intérieur de la paroi du canal (11) est au moins partiellement micro-structurée.

**15.** Inhalateur selon l'une quelconque des revendications 1 à 14, **caractérisé en ce qu'**une résistance à l'écoulement d'air (25) est disposée sur ou devant l'ouverture d'entrée (3).

**16.** Inhalateur selon la revendication 15, **caractérisé en ce que** la résistance à l'écoulement d'air (25) est une plaque perforée comportant au moins un trou.

**17.** Inhalateur selon l'une quelconque des revendications 1 à 16, **caractérisé en ce qu'**il est prévu sur le canal mélangeur (1) une unité de dosage (2) à commande manuelle pour le liquide, avec une plage de volume de 1 à 300 microlitres.

**18.** Inhalateur selon la revendication 17, **caractérisé en ce que** l'unité de dosage (2) présente un agencement à piston-cylindre (36, 38) destiné à exercer une pression sur le liquide (32) à injecter dans le canal mélangeur (1) et une pièce d'actionnement (43) déplaçable par une pression manuelle, dont la course d'actionnement peut être convertie, par un dispositif à ressort (49), en un déplacement relatif entre le piston (38) et le cylindre (36) de l'agencement à piston-cylindre.

**19.** Inhalateur selon la revendication 18, **caractérisé en ce que** le cylindre (36) est inséré de façon étanche dans un dispositif de fermeture (34) d'un réservoir (33) contenant le liquide (32) et pénètre dans le réservoir (33), **en ce qu'**une tige de piston (37) du piston (38) est menée à travers le dispositif de fermeture (34), **en ce que** le piston (38) et la tige de piston (37) sont traversés par un canal de sortie (40) jusqu'à la zone d'injection (6), **en ce que** la tige de piston (37) est fixée dans un boîtier (31) contenant de façon coulissante le dispositif de fermeture (34), **en ce que** la course d'actionnement de la pièce d'actionnement (43) peut être transférée au dispositif de fermeture (34) au moyen du dispositif à ressort (49) contre la force d'un autre dispositif à ressort (41), de telle manière que le dispositif de fermeture (34) soit déplacé par rapport au piston (38), **en ce que** la chambre de pression (53) du cylindre (36) soit en communication, dans l'état inactivé de la pièce d'actionnement (43), avec l'espace intérieur du réservoir (33) par au moins une ouverture (54) dans la paroi du cylindre (36) et par un agencement de soupape (55) et **en ce que** la/les ouverture(s) (54) du cylindre (36) et l'agencement de soupape (55) sont fermés pendant l'actionnement de la pièce d'actionnement (43), après le franchissement de la/des ouverture(s) (54) du cylindre (36) par le piston (38).

Fig. 1

Fig. 2

Fig. 3

Fig. 4

Fig. 5

Fig. 6

Fig. 7

Fig. 8

**Fig. 9**

**Fig. 10**

**Fig. 11**

Fig. 12

Fig. 13

Fig. 14

Fig. 15

α=147°

10

11

6

## Fig. 16

10

α=90°

11

6

## Fig. 17

α=86°

10

6

11

## Fig. 18

α=40°

10

6

11

## Fig. 19

Fig. 20

Fig. 21

# Fig. 22

Fig. 23

## Fig. 24

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0218058 A1
- DE 4306458